# EUROPEAN PATENT APPLICATION

(11) **EP 2 251 008 A1**
(43) Date of publication of application: **17.11.2010**
(21) Application number: 10172237.9
(22) Date of filing: 06.12.2004
(51) Int. Cl.: A61K 31/202, A61K 36/258, A61P 17/00

(54) **Methods for reducing the effects of stress on skin condition**

(30) Priority: 18.12.2003 EP 03257986
(62) Divisional of application: 04803563.8
(71) Applicant: Unilever Plc, A Company Registered In England And Wales under company no. 41424 of Unilever House, London EC4Y 0DY Greater London (GB); Unilever NV, 3013 AL Rotterdam (NL)
(72) Inventor: Barrett, Karen, Elizabeth, Bedford, Bedfordshire MK44 1LQ (GB); Granger, Stewart, Paton, Bebington, Wirral, Merseyside CH63 3JW (GB); Jenkins, Gail, Bedford, Bedfordshire MK44 1LQ (GB); Wainwright, Linda, Jane, Bedford, Bedfordshire MK44 1LQ (GB)
(74) Representative: Acham, Nicholas Clive

(57) **Abstract**

A method is provided for reducing the effects of psychologically-mediated stress on the skin of a human or animal which method comprises administering to the individual a composition capable of inhibiting glucocorticoid-induced chronic stress in a dermal cell or a cell involved in skin inflammatory responses. Also provided are compositions for use in such methods and assay methods for identifying suitable compositions.

## Description

### Field of the invention

The present invention relates to methods of reducing the effects of long-term psychological stress on skin condition. The present invention also relates to compounds for use in such methods and assays for identifying suitable compounds.

### Background to the invention

Neuroendocrine stress resulting from every day life occurrences has been shown, through longitudinal studies, to be a key driver of age-associated conditions. In addition, psychological stress has been shown in several consumer studies to be of concern for individual well being. The impact of this stress on skin appearance and the underlying biological mechanisms are poorly understood

### Summary of the invention

We have developed a model system which mimics the effects of chronic stress and used this system to study the effects of chronic stress on skin. Using this model, we have found that chronic stress, represented as chronic treatment of skin cells with glucocorticoid, leads to a reduction in the ability of skin to respond favourably to acute stress. In particular, we have found that chronically stressed cells have a reduced ability to express proteins required for matrix degradation and matrix synthesis - processes that are needed to repair and maintain skin. In other words, we have found that chronic stress leads to impairment of dermal matrix remodelling which has clear implications with respect to skin condition.

We have also found that chronically stressed skin cells demonstrate a significant increase in sensitivity to acute stress, as demonstrated by an increase in expression of proteins involved in the skin inflammatory response.

These finding have enabled us to develop an assay method for identifying compounds that are capable of reducing the effects of neuroendrocrine-mediated stress, such as psychologically induced stress, on skin condition. Using this assay we have identified a number of agents that reduce the deleterious effects of chronic glucocorticoid exposure on the ability of skin cells to respond to acute stress.

Accordingly, in a first aspect, the present invention provides a method of reducing the effects of psychologically-mediated stress on the skin of a human or animal which method comprises administering to the individual a composition capable of inhibiting glucocorticoid-induced chronic stress in a dermal cell or a cell involved in skin inflammatory responses.

In a related aspect, the present invention provides the use of a composition capable of inhibiting glucocorticoid-induced chronic stress in a dermal cell or a cell involved in skin inflammatory responses in the manufacture of a composition for use in reducing the effects of psychologically-mediated stress on the skin of a human or animal.

Preferably the composition is administered orally or topically.

In a preferred embodiment, the composition comprises a first substance which is capable of inhibiting glucocorticoid-induced chronic stress in a cell involved in skin inflammatory responses and a second substance which is capable of inhibiting glucocorticoid-induced chronic stress in a dermal cell, provided that said first substance and second substance are different.

Preferably, the composition comprises a first substance selected from the group consisting of ginseng Rb1, ginseng Rc, curcumin, 22-OH-cholesterol, ciglitazone, mevinolin, commipheric acid, okadaic acid, liquorice extract and mixtures thereof; and a second substance selected from the group consisting of wolfberry extract, shiitake extract, activin, ginseng Rb1, ginseng Rc, curcumin, ciglitazone, commipheric acid, boswellia extract and mixtures thereof, provided that said first substance and second substance are different

In a second aspect, the present invention provides a composition comprising a first substance which is capable of inhibiting glucocorticoid-induced chronic stress in a cell involved in skin inflammatory responses and a second substance which is capable of inhibiting glucocorticoid-induced chronic stress in a dermal cell, provided that said first substance and second substance are different

In a related aspect, the present invention provides a composition comprising a first substance selected from the group consisting of ginseng Rb1, ginseng Rc, curcumin, 22-OH-cholesterol, ciglitazone, mevinolin, commipheric acid, okadaic acid, liquorice extract and mixtures thereof; and a second substance selected from the group consisting of wolfberry extract, shiitake extract, activin, ginseng Rb1, ginseng Rc, curcumin, ciglitazone, commipheric acid, boswellia extracts and mixtures thereof, provided that said first substance and second substance are different.

In a preferred embodiment, the composition is in the form of a nutritional supplement or a cosmetic composition.

In a third aspect, the present invention provides a method for identifying a compound capable of reducing the effects of psychologically-mediated stress on the skin of a human or animal, which method comprises:
(i) contacting a dermal cell or a cell involved in skin inflammatory responses with a candidate compound in the presence of a glucocorticoid receptor agonist under conditions and for a period of time that would, in the absence of the candidate compound, lead to the cell being chronically stressed;
(ii) subjecting the cell to acute stress;
(iii) analysing one or more cellular markers selected from a marker of inflammatory cell recruitment, where the cell is a cell involved in skin inflammatory responses, a marker of matrix degradation, where the cell is a dermal cell and/or a marker of matrix synthesis in the cell, where the cell is a dermal cell; and
(iv) determining whether the candidate compound affects the status of the one or more cellular markers.

Preferably step (iv) comprises comparing the status of said markers in the presence of the candidate compound with the status of said markers in the absence of the candidate compound.

Preferably the marker of inflammatory cell recruitment is the level of expression of ICAM-1 and/or vCAM-1.

Preferably the marker of matrix degradation is selected from the level of expression of MMP-1, MMP-2 and/or MMP-9.

Preferably the marker of matrix synthesis is selected from the level of expression of procollagen-1, collagen I and/or collagen III.

The present invention also provides a method of producing a composition for reducing the effects of psychologically-mediated stress on the skin of a human or animal which method comprises
(i) contacting a dermal cell or a cell involved in skin inflammatory responses with a candidate compound in the presence of a glucocorticoid receptor agonist under conditions and for a period of time that would, in the absence of the candidate compound, lead to the cell being chronically stressed;
(ii) subjecting the cell to acute stress;
(iii) analysing one or more cellular markers selected from a marker of inflammatory cell recruitment, where the cell is a cell involved in skin inflammatory responses, a marker of matrix degradation, where the cell is a dermal cell and/or a marker of matrix synthesis in the cell, where the cell is a dermal cell;
(iv) determining whether the candidate compound affects the status of the one or more cellular markers;
(v) selecting a candidate compound identified in (iv) as affecting the status of the one or more cellular markers; and
(vi) admixing said compound with a cosmetically or pharmaceutically acceptable carrier or diluent.

### Detailed description of the invention

Unless defined otherwise, all technical and scientific terms used herein have the same meaning as commonly understood by one of ordinary skill in the art.

### Assay methods

The assay method of the present invention can be used to identify compounds that ameliorate the effects of chronic stress on skin. An *in vitro* model is used in which agents are tested for their ability to reduce or prevent the effects of chronically stressing dermal cells, cells involved in skin inflammatory responses, or cells derived therefrom with glucocorticoid (GC) receptors agonists, such as glucocorticoids. In the absence of a test agent, exposure of cells to a GC receptor agonist over a period of time has a deleterious effect on the response to the cells to acute stress, such as oxidative stress. Consequently, the effect of the GC receptor agonist-mediated stress is measured by subjecting the cells to acute stress, following the pre-treatment period, and measuring the status of key markers, in particular markers of inflammation/inflammatory cell recruitment, matrix synthesis and/or matrix degradation.

Suitable cells for use in the assay method fall into two categories: (1) cells involved in inflammatory responses and (2) cells of the dermis.

Cells involved in inflammatory responses in the skin include endothelial cells, adipocytes, immunocytes, mast cells, Langerhans' cells and cells of haemopoietic origin, such as T lymphocytes, macrophages, leucocytes and neutrophils, and cells derived therefrom. Preferred cells are endothelial cells. Cells of the dermis include dermal follicle cells (dermal papilla and connective tissue sheath) dermal fibroblasts, hair follicle keratinocytes and melanocytes, cells of the sebaceous gland, such as sebocytes, and cells derived therefrom. Preferred cells are fibroblasts and melanocytes. Cells are generally of mammalian origin.

Preferred cell types include endothelial cells when measuring markers of inflammatory status and dermal fibroblasts, more preferably neonatal dermal fibroblasts when measuring markers of matrix turnover.

Another preferred cell type is hair follicle melanocytes since these cells are responsible for providing hair pigmentation. Consequently, these cells can be used in the assay method of the invention to identify agents that can be used to reduce the effects of psychological stress on loss of hair colour/hair greying.

Cells may be primary cells that can only be subjected to a limited number of passages in cell culture or immortalised cell lines. The term "derived therefrom" is intended to mean immortalised cell lines derived from primary cell cultures.

Cells are grown and passaged using standard cell culture techniques well known in the art. Culture media are typically supplemented with animal-derived serum products and growth factors as required for specific cell types.

In the first stage of the assay, cells are subjected to pre-treatment with one or more glucocorticoid (GC) receptor agonists, such as glucocorticoids, which bind to and activate a GC receptor. Particular examples of GC receptor agonists include dexamethasone, hydrocortisone, cortisol, prednisalone and betamethasone.

Pre-treatment with one or more GC receptor agonists includes the possibility that the agonists are produced by the cells in response to the administration of another compound, such as corticotrophin releasing hormone (CRH). However, it is preferred that the pre-treatment involves addition to the culture medium of a GC receptor agonist, i.e. direct treatment, rather than stimulation of the cells to produce glucocorticoid *in situ.*

The GC receptor agonist is typically added to the culture medium at a concentration of from 1 nM to 10 µM.

The cells are preferably pre-treated with the GC receptor agonist for at least 2 days, more preferably at least 3 or 4 days, most preferably at least 5 days. Typically, the GC receptor agonist is administered periodically, for example daily, since this is likely to mimic more closely the continual effects of chronic stress.

Candidate agents are added to the culture medium during the pre-treatment stage, generally at the beginning, since the aim is to determine whether the agent can antagonise the effects of the GC receptor agonist. This is conveniently carried out at about the same time as the GC receptor agonist is added. Again, typically, the candidate agent or agents is/are administered periodically, for example daily.

Following pre-treatment, cells are subjected to acute stress and their response to the acute stress determined. Acute stress includes oxidative stress, with phorbol myristate acetate for example, other forms of chemical stress, mechanical stress, or electromagnetic stress, such as UV irradiation.

Tissue culture supernatant and/or cell pellets are harvested at one or more suitable time points and tested to determine the status of the markers of interest. For example, the levels of expression of a gene of interest may be determined using nucleic acid-based detection techniques, e.g. PCR or hybridisation, or protein-based detection techniques such as immunoassays.

Suitable markers of inflammatory status include intercellular adhesion molecules (ICAMs) such as ICAM-1 and vCAM. Suitable markers of matrix synthesis include procollagen-1, collagen I and collagen III, and suitable markers of matrix degradation include MMP-1, MMP-2 and MMP-9 (metallomatrix proteases).

Suitable agents for use in the methods of the invention described below will reduce the levels of the markers of inflammatory status/inflammatory cell recruitment compared to the GC receptor agonist only control, preferably to within at least +20% or +10% of the vehicle only control.

Suitable agents for use in the methods of the invention described below will increase the levels of the markers of matrix synthesis and/or markers of matrix degradation compared to the GC receptor agonist control, preferably to within at least -50% or -20% of the vehicle only control.

Suitable time points for testing the status of the markers of interest will typically vary depending on the specific marker. For example, ICAM-1 expression is preferably measured at least once from between 3 hours to 7 hours post-acute stress. By contrast, MMP-1 expression is preferably measured at least once from between 18 hours to 36 hours post-acute stress.

Candidate agents can include glucocorticoid receptor antagonists, PPAR-γ receptor agonists, AP-1/NF-kB inhibitors, PXR agonist, LXR agonists, cortisol inhibitors and phosphatase inhibitors. In general, combinatorial libraries, peptide and peptide mimetics, defined chemical entities, and natural product libraries may be screened for activity as inhibitors of GC-mediated chronic stress.

Agents for use in the methods of the invention are intended to target the chronic effects of glucocorticoids on skin directly. Consequently, it may be desirable to confirm that the test agents are not directly affecting the expression of the cellular markers of interest during the acute stress treatment step instead of the effects of the GC receptor agonist during the pre-treatment step. This can for example be accomplished by the use of a control that includes the test agent during the pre-treatment step but no GC receptor agonist.

### Methods of reducing the effects of psychologically-mediated stress on skin

The present invention is based on the finding that chronic exposure of skin cells, or cells that are involved in skin inflammatory responses, to glucocorticoids disrupts the mechanisms that enable the skin to cope with acute stress. The implications of this finding is that blocking the deleterious effects of glucocorticoids on skin cells will reduce the effects of psychologically-mediated stress on the skin of humans and other animals. This in turn will reduce or ameliorate the effects of psychological stress on skin condition.

Accordingly, the present invention provides a method of reducing the effects of psychologically-mediated stress on the skin of a human or animal which method comprises administering to the individual a composition capable of inhibiting the effects of glucocorticoid-induced chronic stress on the skin, in particular a composition which inhibits the effects of glucocorticoid-induced chronic stress on the skin's response to acute stress, such as oxidative stress and/or UV exposure. Since we have determined that the effects of glucocorticoid-induced chronic stress on the skin include both a reduction in the skin's matrix turnover and an increase in the inflammatory response to acute stress, in a preferred embodiment, the composition is able to reduce these two distinct effects. This may be by means of a single active ingredient or by two separate ingredients that target each effect separately.

Thus, in a preferred embodiment, the composition comprises a first substance which inhibits glucocorticoid-induced chronic stress in a cell involved in skin inflammatory responses and a second substance which inhibits glucocorticoid-induced chronic stress in a dermal cell, provided that said first substance and second substance are different. Preferably the cell involved in skin inflammatory responses is an endothelial cell. Preferably the dermal cell is a fibroblast.

Matrix turnover can in turn be divided into two processes - matrix degradation and matrix synthesis. Preferably, the composition comprises a substance that reduces the deleterious effects of chronic GC-mediated stress on both processes.

In another aspect, the present invention provides a method of reducing the effects of psychologically-mediated stress on the hair colour of a human or animal which method comprises administering to the individual a composition which inhibits the effects of glucocorticoid-induced chronic stress in a melanocyte, in particular a composition which inhibits the effects of glucocorticoid-induced chronic stress on the cell's response to acute stress.

Compositions may be administered by a variety of routes, such as topically or systemically, e.g. orally. Compositions are typically administered at a frequency of from three times daily to twice weekly.

### Compositions

Compositions of the invention and for use in the methods of the invention preferably comprise a first substance which is capable of inhibiting glucocorticoid-induced chronic stress in a cell involved in skin inflammatory responses and a second substance which is capable of inhibiting glucocorticoid-induced chronic stress in a dermal cell, provided that said first substance and second substance are different.

Such substances include PPAR-γ agonists, AP-1/NF-kB inhibitors, PXR agonists, LXR agonists, cortisol inhibitors and phosphatase inhibitors. Specific examples include ginseng Rb1, ginseng Rc, curcumin, 22-OH-cholesterol, ciglitazone, mevinolin, commipheric acid, okadaic acid, liquorice extract, wolfberry extract, shiitake extract, activin, ginseng Rb1, ginseng Rc and boswellia extract.

Additional active agents can be identified using the assay method of the invention described above. In particular, active agents that reduce the effects of chronic GC mediated stress on the skin's inflammatory response to acute stress can be identified using the assays of the invention that use cells involved in the inflammatory response, testing for markers of inflammatory cell recruitment such as ICAM-1 or vCAM expression. Active agents that reduce the effects of chronic GC mediated stress on the skin's processes of matrix turnover, i.e. matrix degradation and/or matrix synthesis, can be identified using the assays of the invention that use dermal cells, testing for markers of matrix degradation and/or matrix synthesis response such as MMP-1 and/or procollagen-1 expression.

In a preferred embodiment, wherein the composition comprises a first substance selected from the group consisting of ginseng Rb1, ginseng Rc, curcumin, 22-OH-cholesterol, ciglitazone, mevinolin, commipheric acid, okadaic acid, liquorice extract and mixtures thereof; and a second substance selected from the group consisting of wolfberry extract, shiitake extract, activin, ginseng Rb1, ginseng Rc, curcumin, ciglitazone, commipheric acid, boswellia extract and mixtures thereof, provided that said first substance and second substance are different.

### Topical compositions

The compositions of the invention can be administered topically to a subject, i.e., by the direct laying on or spreading of the composition on skin, including the scalp. Such compositions can be prepared by combining a safe and effective amount of an active substance or substances as described above with a pharmaceutically-acceptable topical carrier.

The topical compositions useful in this invention may be made into a wide variety of product types. These include, but are not limited to lotions, creams, gels, sticks, sprays, ointments, pastes, essences, mousses and cosmetics. These product types may comprise several types of carrier systems including, but not limited to solutions, emulsions, gels, dermal patches and solids.

The topical compositions useful in this invention formulated as solutions typically include a pharmaceutically-acceptable aqueous or organic solvent. The terms "pharmaceutically-acceptable aqueous solvent" and "pharmaceutically-acceptable organic solvent" refer to a solvent which is capable of having dispersed or dissolved therein the active(s), and possesses acceptable safety properties (e.g., irritation and sensitisation characteristics). Examples of suitable organic solvents include: propylene glycol, polyethylene glycol (200-600), polypropylene glycol (425-2025), poly vinyl pyrrolidine, propylene glycol-14 butyl ether, glycerol, 1,2,4-butanetriol, sorbitol esters, 1,2,6-hexanetriol, ethanol, isopropanol, butanediol, and mixtures thereof. These solutions useful in this invention preferably contain from about 0.001% to about 10%, more preferably from about 0.01% to about 8% more preferably still from about 0.1 % to about 5%, also preferably from about 0.5% to about 3% of the active, and preferably from about 50% to about 99.99%, more preferably from about 90% to about 99% of an acceptable aqueous or organic solvent.

If the topical compositions useful in this invention are formulated as an aerosol and applied to the skin as a spray-on, a propellant is added to a solution composition.

Topical compositions may be formulated as a solution comprising an emollient, i.e. a material used for the prevention or relief of dryness, as well as for the protection of the skin. A wide variety of suitable emollients are known and may be used herein (see Sagarin, Cosmetics, Science and Technology 2nd Edition, Vol. 1, pp. 32-43 (1972)). Such compositions preferably contain from about 2% to about 50% of a topical pharmaceutically-acceptable emollient.

If the carrier is formulated as an emulsion, preferably from about 1 % to about 10%, more preferably from about 2% to about 5%, of the carrier system comprises an emulsifier. Emulsifiers may be nonionic, anionic or cationic. Suitable emulsifiers are disclosed in, for example, McCutcheon's Detergents and Emulsifiers, North American Edition, pages 317-324 (1986).

Single emulsion skin care preparations, such as lotions and creams, of the oil-in-water type and water-in-oil type are well known in the cosmetic art. Such emulsions can stabilise and enhance the penetration of actives. Multiphase emulsion compositions, such as the water-in-oil-in-water type may also be used. In general, such single or multiphase emulsions contain water, emollients and emulsifiers as essential ingredients.

Another emulsion carrier system that can be used is a micro-emulsion carrier system. Such a system comprises from about 9% to about 15% squalane; from about 25% to about 40% silicone oil; from about 8% to about 20% of a fatty alcohol; from about 15% to about 30% of polyoxyethylene sorbitan mono-fatty acid (commercially available under the trade name Tweens) or other nonionics; and from about 7% to about 20% water.

Liposomal formulations can also be used. These formulations can stabilise actives and also improve delivery of actives which do not penetrate well. Such compositions can be prepared by first combining the active with a phospholipid, such as dipalmitoylphosphatidyl choline, cholesterol and water according to the method described in Mezei & Gulasekharam, Journal of Pharmaceutics and Pharmacology, Vol. 34 (1982), pp. 473-474, or a modification thereof. Epidermal lipids of suitable composition for forming liposomes may be substituted for the phospholipid. The liposome preparation is then incorporated into one of the above topical carrier systems (for example, a gel or an oil-in-water emulsion) to produce the liposomal formulation. Other compositions and cosmetic/pharmaceutical uses of topically applied liposomes are described in Mezei, M., "Liposomes as a Skin Drug Delivery System", Topics in Pharmaceutical Sciences (D. D. Breimer and P. Speiser, eds.), Elsevier Science Publishers B. V., New York, N.Y., 1985, pp. 345-358.

If the topical compositions are formulated as a gel or a cosmetic stick, such compositions can be formulated by the addition of a suitable amount of a thickening agent, as disclosed supra, to a cream or lotion formulation.

Topical compositions may also be formulated as makeup products, such as foundations. Foundations are solution or lotion-based with appropriate amounts of thickeners, pigments and fragrance.

The topical compositions may contain, in addition to the aforementioned components, a wide variety of additional oil-soluble materials and/or water-soluble materials conventionally used in topical compositions, at their art-established levels.

Various water-soluble materials may also be present in the compositions. These include humectants, proteins and polypeptides and preservatives. In addition, the topical compositions useful herein can contain conventional cosmetic adjuvants, such as dyes, opacifiers (e.g., titanium dioxide), pigments and perfumes.

The topical compositions useful in this invention may also include a safe and effective amount of a penetration enhancing agent. A preferred amount of penetration enhancing agent is from about 1% to about 5% of the composition. Examples of useful penetration enhancers are described in US 6,068,834. Other conventional skin care product additives may also be included in the compositions. For example, collagen, hyaluronic acid, elastin, hydrolysates, primrose oil, jojoba oil, epidermal growth factor, soybean saponins, mucopolysaccharides, and mixtures thereof may be used.

Various vitamins may also be included in the compositions. For example, Vitamin A, and derivatives thereof, Vitamin B2, biotin, pantothenic, Vitamin D, and mixtures thereof may be used.

It may be desirable to include in the compositions of the invention, one or more sun screening agents. A wide variety of conventional sun screening agents are disclosed in, for example, Cosmetics, Science and Technology 2nd Edition (1972), Vol. 1, Chapter VIII, pages 189 et seq. See also US 6,068,834.

The sun screening agent must be compatible with the active(s). The composition preferably comprises from about 1% to about 20%, more preferably from about 2% to about 10%, of a sun screening agent. Exact amounts will vary depending upon the sunscreen chosen and the desired Sun Protection Factor (SPF).

An agent may also be added to any of the compositions of the invention to improve the skin substantivity of those compositions, particularly to enhance their resistance to being washed off by water, or rubbed off. A preferred agent which will provide this benefit is a copolymer of ethylene and acrylic acid. Compositions comprising this copolymer are disclosed in U.S. 4,663,157.

The present invention relates to methods of reducing the effects of psychologically-mediated stress on the skin of a human or animal. Such methods comprise the administration of a safe and effective amount of a composition of the invention to the skin or regions thereof the skin, such as the scalp or face. The amount of active agent and frequency of application will vary depending on the initial condition of the skin.

Any dose which is less than the toxic level may be used, thus it is contemplated that for certain dosage forms, particularly topical dosage forms, the "dose" is any amount that provides the desired effect, and that amount may be so large due to frequency of application and amount applied that the maximum effective amount is irrelevant.

A safe and effective amount of active in a topical composition is applied, generally from about 1 µg to about 1 mg per cm² skin per application, preferably from about 2 µg to about 800 µg/cm² skin per application, more preferably from about 30 µg to about 700 µg/cm² skin, most preferably from about 75 µg to about 250 µg/cm² skin. Frequency of application typically ranges from about four times a day to about twice a week, more preferably from about three times a day to about once every other day, more preferably at least twice daily.

### Pharmaceutical compositions

Compositions of the invention can be combined with a pharmaceutically acceptable carrier or diluent to produce a pharmaceutically composition. Pharmaceutically acceptable diluents or carriers suitable for use in such compositions are well known in the art of pharmacy. The compositions of the invention typically contain from 1 to 90% by weight of active, such as from 1 to 50% by weight of active.

The pharmaceutical composition may consist of solid dosage forms such as tablets, hard gelatin capsules, soft gelatin capsules, bulk powders, and microcapsules of the drug. Alternately, it may consist of a liquid dosage form such as an aqueous or nonaqueous solution, emulsion, or suspension.

Solid compositions for oral administration are preferred compositions of the invention. Solid compositions of the invention are preferably prepared in unit dosage form, such as in the form of tablets and capsules. Suitably tablets may be prepared by mixing the active combination with an inert diluent such as calcium phosphate in the presence of disintegrating agents, for example maize starch, and lubricating agents, for example magnesium stearate, and tableting the mixture by known methods. Such tablets may, if desired, be provided with enteric coatings by known methods, for example by the use of cellulose acetate phthalate. Similarly, capsules, for example hard or soft gelatin capsules, containing the active combination optionally in the form of beads with or without added excipients, may be prepared by conventional means and, if desired, provided with enteric coatings in a known manner. The tablets may be formulated in a manner known to those skilled in the art so as to give a controlled release of the compound of the present invention.

Controlled release forms of the pharmaceutical compositions of the present invention include rapid release formulations such as soluble granules or melt filled fast release capsules, delayed release formulations such as tablets provided with enteric coatings, for example, of cellulose acetate phthalate and, in particular, sustained release formulations. Numerous types of sustained release formulations are known to those skilled in the art. Typically, the active combination may be encapsulated within a release retarding coating, for example, a copolymer of cellulose ether and acrylate, or may be bound to small particles such as, for example, ion exchange resin beads. Alternatively, the active combination may be incorporated into a matrix containing a release retarding agent such as a hydrophilic gum e.g. xanthan gum, a cellulose derivative eg. hydroxypropyl methylcellulose, or a polysaccharide, wax or plastics material.

The active combination may be formulated into a solid dosage form in which the two active drugs are kept separate. For example, the dosage form may be a bilayer tablet in which the active drugs are contained in different layers. The different layers can be formulated so as to provide the optimum release profile for each drug.

Liquid fill compositions for example viscous liquid fills, liquid paste fills or thixotropic liquid fills are also suitable for oral administration. Melt filled compositions may be obtained by mixing the active combination with certain esters of natural vegetable oil fatty acids, for example, the Gelucire TM range available from Gattefosse to provide a variety of release rates. Suitably a melt-filled capsule comprises from 10 to 80% active and from 20 to 90% of a fatty acid ester excipient which comprises one or more polyol esters and triglycerides of natural vegetable oil fatty acids.

Preferably oral liquid compositions comprise from 1 to 10 wt% active together with from 1 to 50 wt% of a diluent, the remainder made up with sterile water. Optionally the composition may contain suspending agents, thickeners, cosolvents such as alcohol and/or preservatives. Suitable diluents include sweetening agents for example sorbitol, xylitol or sucrose. Suitable suspending agents or thickeners include cellulose gums, agar or natural gums, for example xanthan gum. Flavourings or other taste-masking agents known to those skilled in the art for example saccharin, sodium saccharin, acesulpham K or aspartame may be added.

Compositions of the invention suitable for parenteral administration can be prepared by combination of the active with known pharmaceutical forms for such administration, for example sterile suspensions or sterile solutions of the active in a suitable solvent such as saline.

The preferred modes of administration are orally, topically, and parenterally (for example, by subcutaneous injection, intramuscular injection, intra-articular injection, intravenous injection and the like). Thus, specific modes of administration include, without limitation, oral, transdermal, mucosal, sublingual, intramuscular, intravenous, intraperitoneal, and subcutaneous administration, as well as topical application. The most preferred method of application is topical or oral.

The amount of the compound administered depends upon the bioavailability of the compound from the pharmaceutical composition, in particular where oral administration is used. Typically, however, the compounds of this invention are dosed in an amount of from about 0.01 mg/kg of body weight to about 100 mg/kg, preferably from about 0.1 to about 30 mg/kg of body weight. The amount of the pharmaceutical composition depends upon the percent of compound within its formula, which is a function of the amount of the compound required per dose, its stability, release characteristics and other pharmaceutical parameters.

The preferred method of injectable administration depends upon the solubility and stability of the particular active being used.

The routes of administration and dosages described are intended only as a guide since a skilled practitioner will be able to determine readily the optimum route of administration and dosage for any particular patient and condition.

Another means of systemic dosing comprises dosing any of the aforementioned compositions in a food product which does not therefore necessarily require use of a pharmacologically acceptable carrier.

As used herein, the term "food products" includes both food products as such and beverages. Suitable food products as such include spreads, dairy products (including milk and yoghurts), desserts, convenience foods/snacks, breakfast cereals and cereal bars, ready-cook meals, bread and frozen confections such as ice creams, water ices and sorbets and yoghurt ice creams. Food products also include dietary/nutritional supplements. Suitable beverages include tea, tea-flavoured drinks, coffee, soft drinks (e.g. carbonated squashes etc) and fruit juice.

The food products are typically supplemented with the active ingredient(s) of the invention so that they contain higher amounts of the active ingredient(s) than they would normally contain.

The present invention will now be described further with reference to the following examples which are illustrative only and non-limiting.

The examples refer to the following figures:
Figure 1 - a graph showing levels of ICAM-1 expression in HUVECs.
Figure 2 - a graph showing levels of ICAM-1 expression in HMVEC-d cells.
Figure 3 - a graph showing levels of ICAM-1 expression in HUVECs.
Figure 4 - a graph showing levels of ICAM-1 expression in HUVECs.
Figure 5 - a graph showing levels of MMP-1 expression in human neonatal dermal fibroblast cells.
Figure 6 - a graph showing levels of MMP-1 expression in human adult-derived dermal fibroblast cells.
Figure 7 - a graph showing levels of MMP-9 expression in human neonatal dermal fibroblast cells.
Figure 8 - a graph showing levels of procollagen-1 expression in human neonatal dermal fibroblast cells.
Figures 9 to 13 - graphs showing levels of ICAM-1 expression in HUVECs.

### EXAMPLES

### Example 1 - Effect of chronic stress on the inflammatory status of endothelial cells Outline of Experimental Approach

An *in vitro* model has been developed to investigate the impact of chronic stress on the inflammatory status of endothelial cells.
1. Cells are grown in 6-well (9.5cm²) plates.
2. Synthetic glucocorticoid (Dexamethasone) pre-treatment is added daily for 5 days to 'chronically stress' the cells.
3. Tissue culture supernatant and cell pellets are harvested at timepoint T0.
4. The cells are oxidatively stressed with 1 µM Phorbol Myristate Acetate (PMA).
5. Tissue culture supernatant and cell pellets were harvested at 6, 24 and 48 hours (T6, T24 and T48, respectively) post-PMA treatment.
6. All tissue culture supernatant was assayed for Lactate Dehydrogenase (LDH), as a measure of cytotoxicity, and Interleukin-6 synthesis.
7. All cells were counted (Beckman Coulter Counter) and pelleted and cell lysate assayed for ICAM-1 expression (Intra Cellular Adhesion Molecule 1).

### Materials and Methods

### Culture of Endothelial Cells

HUVEC cells (Human umbilical vein endothelial cells, TCS Biologicals) were cultured and passaged in EGM-2 (Endothelial growth medium, Biowhittaker) supplemented with heparin, VEGF (vascular endothelial growth factor), gentamicin sulphate, ascorbic acid, HEGF (Human endothelial growth factor), hydrocortisone, HFGF-B (Human fibroblast growth factor B), R3-IGF-1 (long R insulin-like growth factor 1) and FBS (foetal bovine serum).

HMVEC-d cells (Human dermal microvascular endothelial cells, Clonetics) were cultured and passaged in EGM-2 MV (Microvascular endothelial growth medium, Biowhittaker) supplemented with VEGF (vascular endothelial growth factor), gentamicin sulphate, ascorbic acid, hydrocortisone, HFGF-B (Human fibroblast growth factor B), R3-IGF-1 (long R insulin-like growth factor 1) and FBS (foetal bovine serum).

Cells were routinely plated out in 6-well tissue culture dishes, at a seeding density of ∼5000 cells/cm² in 2ml complete medium/well, 48 hours before starting the experiment, and incubated at 37°C in 5% CO₂.

### Addition of Test Solutions

Pre-treatment and test solutions were prepared in EGM-2 containing all supplements except hydrocortisone.

Endothelial cells were pre-treated daily for a period of five days with either 1 :M Dexamethasone (a synthetic glucocorticoid, Sigma D8893) or 1 to 100 nM CRH (Corticotrophin Releasing Hormone, Calbiochem 05-23-0050). Following this, the cells were oxidatively stressed for 24 hours with 1 µM PMA (Sigma P8139).

Receptor antagonists were added to the cells during the pre-treatment period. These included the Glucocorticoid Receptor Antagonist; 2 µM Mifepristone (Sigma M8046) and two CRH receptor antagonists; 200 nM Astressin (Sigma A4933) and 200 nM "-helical CRF peptide antagonist (Sigma C2917).

The effects of acute Dexamethasone and recombinant CRH were also investigated by pre-treating the endothelial cells with 0.1 % ethanol vehicle daily for five days followed by the addition of PMA together with either glucocorticoid or CRH.

### Harvesting Samples and Cell Number

Any change in cell morphology was noted before the cells were harvested. Both the tissue culture supernatant and the endothelial cells were harvested after the five-day pre-treatment period (TO), at 6 hours and 24 hours after addition of PMA (T6 and T24) and at 24 hours after removal of the PMA (T48). All tissue culture supernatants were stored at -20°C.

1 ml of trypsin/EDTA solution (Invitrogen 25300-054) was added to each well, and the plate incubated at 37°C until the cells detached. 50:1 of this cell suspension was added to 9.95mls of Isoton II (Beckman Coulter) in an accuvette and 0.5ml of this suspension was counted twice in a Coulter Particle Counter Z1 with 140:m aperture.

The remaining 950 µl of original cell suspension was centrifuged at 13000 rpm in a microcentrifuge for 10 minutes. The supernatant was discarded and the cell pellet washed with 500 µl of Dulbecco's PBS and centrifuged as before. The supernatant was discarded as before and cell pellet stored at -20°C prior to cell lysis. The number of cells per pellet was estimated from the Coulter Counter data.

### Cytotoxicity Assay (Promega)

All tissue culture supernatant was examined for cytotoxicity using the Promega CytoTox 96 non-radioactive cytotoxicity assay. This assay quantitatively measures lactate dehydrogenase (LDH) released upon cell lysis and is a good indication of cell viability. 50 µl of tissue culture supernatant or control medium was added to duplicate wells of a 96-well microtitre plate. 50 µl of CytoTox reagent was added and mixed well. The plate was incubated in the dark, at room temperature, for 30 minutes. After this time 50 µl of stop solution was added to each well and the absorbance of the plate was read at 492nm. Any test samples giving an absorbance value of more than double that of the control medium was considered to be cytotoxic. No results have been included from samples that showed any signs of cytotoxicity.

### Preparation of Cell lysate

All cell pellets were lysed on ice for 30 minutes in 1 ml cell lysis buffer per 2.5 x 10⁶ cells. The lysis buffer contained 1% NP-40, 0.1 % sodium deoxycholate, 0.1 % SDS, 6 mM sodium chloride and 0.05M Tris at pH 7.6. Protease inhibitor cocktail (1000X; Sigma P8340) was added prior to use at a level of 10 µl per ml of lysis buffer. The partially lysed cell pellets were completely homogenised with a pellet pestle and unwanted cell debris removed by centrifugation for 20 minutes at 20,000g at 4°C. The clarified cell lysate was frozen at -80°C until needed.

### Total Protein Assay (Pierce)

The total protein concentration of each cell lysate was measured using the Pierce BCA protein assay kit. A set of eight standard solutions ranging from 0 to 1200 µg/ml protein was prepared from the supplied 2 mg/ml BSA stock solution. 10 µl of standard or cell lysate was added to duplicate wells of a flat-bottomed, 96-well microtitre plate. The reagent solution was prepared according to the kit instructions from 50 parts reagent A and 1 part reagent B. 200 µl of the final reagent was added to each well of the microtitre plate. The plate was mixed, covered and incubated at 37°C for 30 minutes and absorbance read at 562 nm. A protein standard curve was constructed and used to determine the protein concentration of each cell lysate.

### ICAM-1 ELISA (R&D Systems)

ICAM-1 protein in each cell lysate was estimated using the Human sICAM-1 DuoSet ELISA kit (R&D Systems DY720) according to the manufacturer's instructions.

The capture antibody was diluted to a final concentration of 4 µg/ml in PBS and 100 µl was used to coat each well of a 96-well microtitre plate overnight at room temperature. The plate was then washed three times with wash buffer (0.05% Tween 20 in PBS). Each well received 300 µl of blocking buffer (1% BSA, 5% sucrose and 0.05% sodium azide in PBS), and the plate was incubated at room temperature for 1 hour before being washed as before. Each cell lysate was then diluted 1/200 in reagent diluent (1 % BSA in PBS) and 100 µl added to duplicate wells of the antibody coated plate. Eight ICAM-1 standards were prepared in reagent diluent, at concentrations ranging from 0 to 1000 pg/ml, and duplicate 100 µl standards were added to the appropriate wells on the plate. A separate set of standards was routinely used for each plate. The plate was incubated at room temperature for 2 hours before being washed again. 100 µl of detection antibody, diluted to a final concentration of 100 ng/ml, was added to each well and the plate incubated at room temperature for 2 hours. The plate was washed as before. Each well received 100 µl of streptavidin-HRP conjugate diluted1/200 in reagent diluent and the plate incubated at room temperature, in the dark, for 20 minutes. The plate was washed for the final time and 100 µl of substrate solution (1:1 mixture of colour reagent A and colour reagent B, R&D Systems DY999) was added to each well. After 20 minutes incubation, at room temperature in the dark, the colour development was stopped by the addition of 50 µl of 2N sulphuric acid. The absorbance of the plates was measured at 450nm with the correction wavelength set at 570nm.

A standard curve was plotted of mean absorbance versus ICAM-1 concentration and the line of best fit calculated by regression analysis. The unknown concentration of ICAM-1 in the samples was calculated from this, taking the lysate dilution factor into account.

To normalise for differences in cell number and total protein concentration, the final result was expressed as ng ICAM-1 per mg of total protein.

### Interieukin-6 ELISA (R&D Systems)

The IL-6 protein concentration of each tissue culture supernatant was assayed using the QuantiGlo Q6000 Human IL-6 assay (R&D Systems) according to the manufacturer's instructions.

Six IL-6 standards were prepared in calibrator diluent at concentrations ranging from 0 to 3000 pg/ml. 50 µl of assay diluent and 150 µl of tissue culture supernatant or standard was added to duplicate wells. The plate was incubated at room temperature for 2 hours on a horizontal orbital plate shaker before being washed four times with wash buffer. 200 µl of IL-6 conjugate was added to each well and the plate incubated at room temperature for 3 hours on a horizontal orbital plate shaker (∼500rpm). The plate was washed as before. Each well received 200 µl of substrate solution and the plate incubated at room temperature, on the benchtop, for 40 minutes. The relative light unit (RLU) of each well was determined using a luminometer set with 1 minute lag time, 1 second/well read time, summation mode and automatic gain on.

A standard curve was plotted of mean RLU versus IL-6 concentration and the line of best fit calculated by regression analysis. The unknown concentration of IL-6 protein in all the samples was estimated from this.

### Results

### Effect of Chronic 1 µM Glucocorticoid Treatment on ICAM-1 synthesis in HUVECs followed by Oxidative Stress with 1 µM PMA

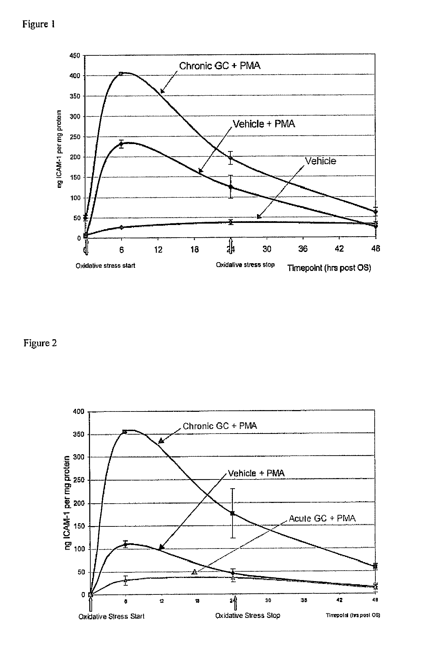

Chronically stressed endothelial cells were oxidatively stressed with 1 µM PMA (as described in the methods and materials) and cells harvested at the given time points to measure changes in ICAM-1 expression (t0, t6, t24 and t48). This additional, acute stress (24 hours) was employed as a mimic of an injury and/or insult to the skin, e.g. UV irradiation. A significantly greater induction in ICAM-1 synthesis and secretion was observed when directly compared to the control, untreated cells (see Figure 1). Further, the induction of ICAM-1 synthesis was significantly greater in cells than had been subjected to glucocorticoid treatment prior to oxidative stress than cells that had not been subjected to glucocorticoid treatment prior to oxidative stress.

This induction of ICAM-1 is believed to reflect a higher degree of inflammatory cell recruitment in the skin.

These experiments were repeated with HMVEC-d cells. Similar results were obtained.

### Effect of Acute 1 µM Glucocorticoid Treatment on ICAM-1 synthesis in HUVECs during Oxidative Stress with 1 µM PMA

We have examined the effect of 'acute stress' in the endothelial cell model. Essentially, synthetic glucocorticoid was added, at the same time as addition of the PMA, to endothelial cells that had been pre-treated (for 5 days) with vehicle alone. Acute GC treatment effectively suppressed the post oxidative stress-induction of ICAM-1 expression, thus reducing the inflammatory status in endothelial cells.

These experiments were repeated with HMVEC-d cells. Similar results were obtained.

### Effect of GC Receptor Antagonist (2 µM Mifepristone) on ICAM-1 synthesis during Chronic 1 µM Glucocorticoid Treatment of HUVECs followed by Oxidative Stress with 1 µM PMA.

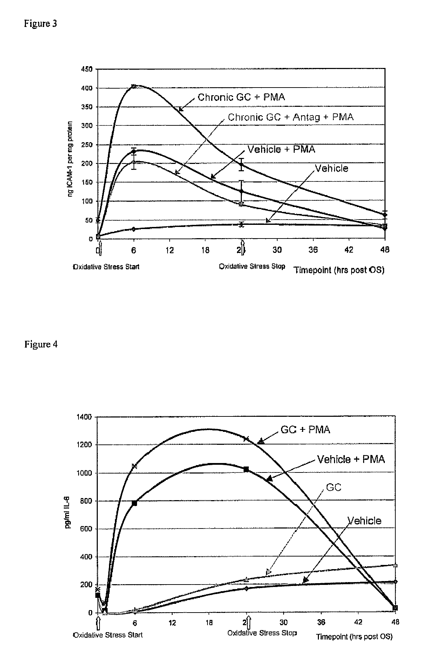

When a GC receptor antagonist (Mifepristone) was added to the cells during the chronic GC pre-treatment step, there was a significant reduction in the oxidative stress-induced increase in ICAM-1 expression following treatment with PMA, to below that seen in cells that had not received chronic GC treatment (Figure 3). These observations suggest that the stress-induced increase in ICAM-1 expression is mediated through the GC receptor.

These experiments were repeated with HMVEC-d cells. Similar results were obtained.

### Effect of corticotrophic releasing hormone Treatment on ICAM-1 synthesis in HUVECs during Oxidative Stress with 1 µM PMA

Stress results in a potent activation of cutaneous corticotrophic releasing hormone (CRH), followed by an induction, via POMC and all its peptide derivatives, of corticosteroids. CRH is one of the key, primary peptides which controls the induction and initiation of the Hypothalamus-Pituitary-Adrenal (HPA) axis in the skin. We investigated whether chronic treatment of HUVECs with CRH would result in a similar exacerbation of ICAM-1 levels as observed in chronically GC-treated cells.

Recombinant CRH was added to the endothelial cells, every day for a period of 5 days. Then, as before, both treated and untreated cells were oxidatively stressed and cells were harvested at the respective time points over 24 hours.

Chronic treatment of endothelial cells with recombinant CRH resulted in a significant increase in ICAM-1 secretion, indicating a higher degree of inflammation (data not shown). However, the increase in ICAM-1 synthesis was seen to be consistently greater in the GC-treated cells that in the CRH-treated cells.

Inclusion of CRH receptor antagonists during the CRH pre-treatment step (astressin; "-helical CRF peptide antagonist), only partially blocked the increased induction of ICAM-1 post-oxidative stress in endothelial cells chronically treated with CRH (data not shown). The levels of ICAM-1 synthesised by these cells were reduced to approximately half of that induced by oxidative stress after pre-treatment with CRH for five days. Surprisingly, synthesis of ICAM-1 was not brought back down to basal levels as previously observed with the glucocorticoid receptor antagonist (mifepristone). Mifepristone completely blocked the increased secretion of ICAM-1 induced by oxidative stress with PMA in endothelial cells chronically treated with GC (see Figure 3). This partial blocking effect may be due to ineffective receptor antagonists or, in fact, that the CRH receptor may not be primarily responsible for the induction of ICAM-1. It may be possible that recombinant CRH, via its stimulation of GC's, results in a combined CRH/GC response. Titration of GC receptor antagonists may suppress these ICAM-1 levels even further.

### Changes in IL-6 Expression by Human Umbilical Vein Endothelial Cells

We have observed an increase in interleukin-6 synthesis and secretion in the GC-treated chronically stressed endothelial cells when compared to vehicle treated cells following oxidative stress with PMA. The vehicle or glucocorticoid alone did not cause increased secretion of IL-6.

### Discussion

We have clearly demonstrated that addition of recombinant glucocorticoid displays a notably divergent effect on PMA-stimulated ICAM-1 expression in endothelial cells. Our results have described, for the first time, how a chronic treatment of GC in endothelial cells significantly increased inflammatory status in comparison to control, untreated cells. However, in contrast, an acute does of GC significantly suppressed the inflammatory status. We propose that the dramatically different responses of endothelial cells to acute and chronic GC treatment may be caused by production of an increasingly dysfunctional local HPA axis within the skin. Typically high levels of inflammation in the skin may result in the production and stimulation of proinflammatory cytokines and proteases which may all contribute to a deterioration in skin condition. This working hypothesis was further validated by similar observations following acute and chronic CRH treatment of endothelial cells.

IL-6 is one of many mediators of the immune system, which help to modulate the function of the HPA axis. It is often released as a consequence of a cascade-like production of IL-1 alpha and TNF-alpha. Surprisingly, we were unable to detect these pro-inflammatory cytokines in the supernatant of GC-treated or untreated endothelial cells (data not shown). The data presented in this report suggests that it may be through activation of IL-6 (the end product of this cascade) that the immune system serves to influence HPA axis activity, by increasing secretion of CRH (and ultimately GC levels), in a dose dependent manner. In this report, we have demonstrated that chronically GC-treated endothelial cells have significantly higher levels of IL-6 in comparison to the control, untreated cells. This data would also be in keeping with our proposal that these chronically stressed endothelial cells have a defective HPA axis, where changes in the responsivity of the feedback loop would prevent downregulation of IL-6 (via the interaction between GC and AP-1).

In conclusion, we believe that this model of inflammation will prove invaluable in our understanding of the changes in the local HPA axis in the skin, associated with chronic insults. Our ongoing studies indicate that chronic GC treatment results in the downregulation of GC receptor levels, thus producing a defective feedback loop within the skin. We have gone on to test a range of nutritional agents in this model to see if we can alleviate the induction of inflammatory cell recruitment, as described in Example 3.

### Example 2 - Effect of chronic stress on dermal matrix remodeling

The dermal matrix component of the skin is essentially composed of elastin fibres, collagen fibrils, protein polysaccharides and other glycoproteins. This complex of connective tissues serves as a scaffold for the dermis, to which cells (like fibroblasts) may attach. The major protein component of these tissues is collagen, a fibrous protein of heterogeneous nature. Collagen Type I is the dominant structural protein in skin and its destruction is a major contributor to the appearance of aged skin. Normal fibrillogenesis requires multiple post-translational modifications, which include the proteolytic conversion of precursor procollagen to collagen. The amount of procollagen I estimated might therefore reflect the amount of collagen I molecules being synthesised. In fact, the histological characteristics of a damaged, aged skin typically include compressed, hardened, disordered bundles of collagen fibres. A young, healthy skin retains the ability to degrade and remove this dense, solid mass of disorganised matrices.

Matrix degradation is hence a considerable factor in dermal matrix remodelling, and the release of dermal proteases, such as matrix metalloproteinase-1 (MMP-1), can help remove aggregated, disorganised collagen fibres from the skin. The MMPs are a large family of over 25 members responsible for degrading connective tissue (reviewed in Woessner, 1994, Ann N Y Acad Sci732: 11-21). They are structurally related endopeptidases that mediate degradation of different macromolecular components of the extracellular matrix and the basement membrane and can be classified into four different subfamilies; the collagenases, the gelatinases, the stromelysins and the membrane MMPs. Human skin expresses a wide range of MMPs, but only MMP-1 (collagenase) has been shown to be capable of attacking native fibrillar collagen. Furthermore, MMP-1 has been shown to be expressed by both keratinocytes and fibroblasts, and therefore, plays a major role in the maintenance of normal collagen turnover and matrix remodelling during wound healing.

In addition, exposure to ultraviolet irradiation (UVR) has also been demonstrated to induce MMP-1 in human skin *in vivo.* MMP-1, therefore, has long been considered to be the principal initiator of collagen breakdown in the skin. UVR has also been shown to induce both MMP-2 (Gelatinase A) and MMP-9 (Gelatinase B); enzymes that are capable of further degrading the fragments produced by collagenolytic enzymes. In summary therefore, release of MMP-1 should cleave native collagen fibres into smaller fragments, which may then be further degraded by gelatinases, MMP-2 and MMP-9. It is believed that the MMPs behave as the chief mediators of connective tissue damage in skin exposed to UVR.

In this study, to understand better the effect of chronic stress on dermal matrix remodelling in the skin, we have therefore measured changes in markers of dermal matrix degradation (MMP-1) and dermal matrix synthesis (Procollagen I) in chronically glucocorticoid-treated primary human dermal fibroblasts, following acute stress with PMA. The experimental approach is similar to that described in example 1 except that the cells tested are dermal fibroblasts and cells are assayed for expression of procollagen-1, MMP-1 and MMP-9 expression.

### Materials and Methods (where different from example 1)

### Culture of Dermal Cells

Primary human dermal fibroblast cells were cultured and passaged in DMEM (Gibco) supplemented with 10% FBS (foetal bovine serum). Cells were routinely plated out in 6-well tissue culture dishes, at a seeding density of ∼5000 cells/cm² in 2ml complete medium/well for 24 hours, and incubated at 37°C in 5% CO₂. Media was removed and cells grown in DMEM & 1% FBS, 24 hours prior to treatments.

### Addition of Test Solutions

Pre-treatment and test solutions were prepared in DMEM containing low serum (1% FBS). Dermal fibroblasts were pre-treated daily for a period of five days with 1 µM Dexamethasone (a synthetic glucocorticoid; Sigma D8893). Following this, the cells were oxidatively stressed for 24 hours with 1 µM PMA (Sigma P8139).

### Human Active MMP-1 and MMP-9 ELISA (R&D Systems)

MMP-1 and MMP-9 protein in each cell supernatant was estimated using the Human active MMP-1 and MMP-9 Fluorokine enzyme kit (R&D Systems F1M00 and F9M00, respectively), according to the manufacturer's instructions.

Six MMP-1 and MMP-9 standards were prepared in calibrator diluent at concentrations ranging from 0.39 to 12.5 ng/ml and 0.25 to 16ng/ml, respectively. 100 µl of assay diluent and 150 µl of tissue culture supernatant or standard was added to duplicate wells. The plate was incubated at room temperature for 3 hours on a horizontal orbital plate shaker before being washed four times with wash buffer. 200 µl of APMA was added to each well and the plate incubated at 37°C for 2 hours in a humidified environment and protected from the light. The plate was washed as before. Each well received 200:1 of substrate solution and the plate incubated at 37°C for 17-20 hours in a humidified environment and protected from the light. The relative fluorescence unit (RFU) of each well was determined using a fluorescence plate set with 1 x 20mS integration time, plate speed ∼ 6; excitation wavelength set to 320nm and emission wavelength set to 405nm.

A standard curve was plotted of mean RFU versus MMP-1 and MMP-9 concentration (respectively), and the line of best fit calculated by regression analysis. The unknown concentration of either active MMP-1 or MMP-9 protein in all of the samples was estimated from this.

### Procollagen I C-Peptide EIA KIT (Takara Bio Inc.)

Collagen I is synthesised as a precursor molecule, Procollagen I. The amount of free propeptide therefore, reflects stoichiometrically, the amount of collagen I synthesised. The Procollagen Type I C-peptide Enzyme Immunoassay (EIA) kit allows for the quantitative determination of Procollagen Type I C-peptide (PIP).

Eight PIP standards were prepared in sample diluent at concentrations ranging from 0 to 640 ng/ml. 100 µl of antibody-Peroxidase conjugate solution and 20 µl of cell lysate (1 µg protein) or standard was added to duplicate wells. The plate was sealed and incubated at 37°C for 3 hours before being washed four times with 400 µl of PBS. Each well then received 100 µl of substrate solution and the plate incubated at room temperature, on the benchtop, for 15 minutes. After this period, 100 µl stop solution was added to each well and absorbance measured at 450nm with a plate reader.

A standard curve was plotted of mean absorbance versus PIP concentration and the line of best fit calculated by regression analysis. The unknown concentration of PIP in all the samples was estimated from this.

### Results

### Changes in MMP-1 Expression in Neonatal-derived human dermal fibroblasts

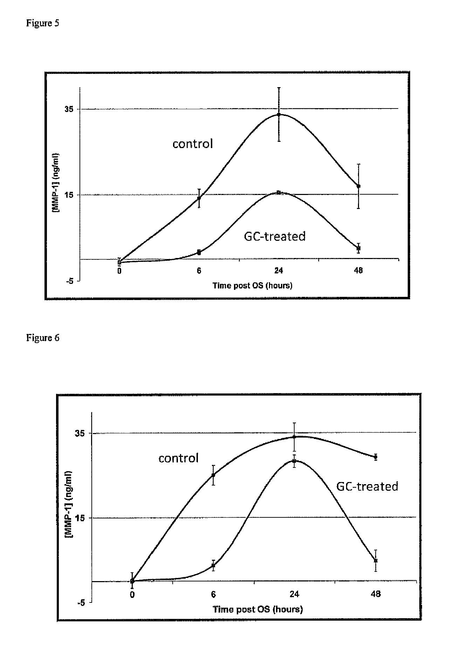

Chronically stressed human neonatal dermal fibroblast cells were oxidatively stressed with 1 µM PMA (as described in the methods and materials), and cells harvested at the given time points to measure changes in MMP-1 expression (T0, T6, T24 and T48). This additional, acute stress (for a period of 24 hours only) was employed as a mimic of an injury and/or insult to the skin (such as UVR). A significant suppression and inhibition of MMP-1 synthesis and secretion was observed when directly compared to the control, untreated cells (∼60% @ T6; 80% @ T24; 70% @ T48) - see Figure 5.

This suppression of MMP-1 is likely to reflect an inability to degrade the dermal matrix in the skin (in particular, collagen type I fibrils).

### Changes in MMP-1 Expression in Adult-derived human dermal fibroblasts

Chronically stressed adult dermal fibroblast cells were also oxidatively stressed with 1 µM PMA, and cells harvested at the given time points to measure changes in MMP-1 expression (T0, T6, T24 and T48). As observed in neonatal-derived fibroblasts, a suppression and inhibition of MMP-1 synthesis was apparent when directly compared to the control, untreated cells (∼85% @ T6; 10% @ T24; 80% @ T48) - see Figure 6.

It is known that MMP-1 levels in the skin increase as a function of chronological age therefore the significantly greater levels of active MMP-1 observed in GC-treated, adult-derived fibroblasts compared with those observed in GC-treated neonatal-derived fibroblasts are not surprising (∼25 ng/ml in GC-treated adult fibroblasts v. 15 ng/ml in GC-treated neonate fibroblasts). Furthermore, it appears that total levels of active MMP-1 in the control, untreated adult dermal fibroblasts may have saturated the monoclonal antibody in the Fluorokine MMP-1 ELISA (NB. Throughout these studies, no ELISA data has shown [MMP-1] to be greater than ∼35ng/ml).

Following these observations, all subsequent experiments were carried out in neonatal-derived fibroblasts.

### Changes in MMP-9 Expression in Neonatal-derived human dermal fibroblasts

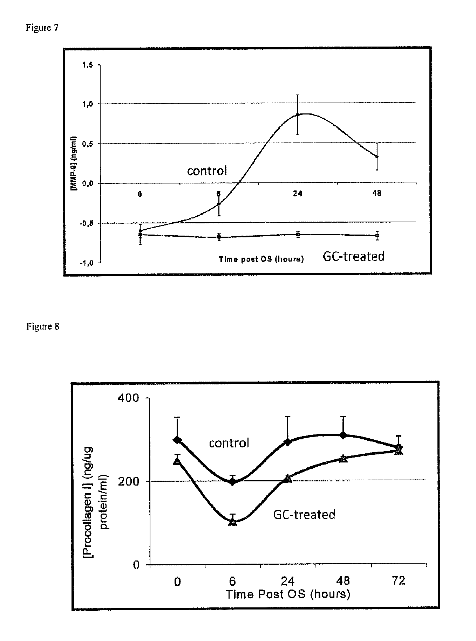

MMP-9 has previously been shown to be one of the most highly effective proteases to help clear MMP-1-generated collagen fragments in the skin. We therefore measured changes in MMP-9 synthesis in this model. An identical pattern to MMP-1 was observed: that is, a significant suppression and inhibition of MMP-9 synthesis and secretion was detected in the chronically stressed dermal fibroblasts when directly compared to the control, untreated cells (figure 7). Whilst levels of MMP-9 proved to be significantly lower than MMP-1, a similar pattern of expression was consistently seen.

### Changes in IL-6 expression in Neonatal-derived human dermal fibroblasts

We observed a significant inhibition in Interleukin-6 synthesis and secretion in the GC-treated chronically stressed dermal fibroblast cells, when compared to vehicle treated cells following oxidative stress with PMA (∼80% reduction in synthesis at T6 and T24) - data not shown.

### Changes in Procollagen I synthesis in Neonatal-derived human dermal fibroblasts

Type I collagen, the major structural component of the skin, is synthesised as a precursor molecule called procollagen I, and large extra domains known as propeptides are cleaved off enzymatically. We have therefore examined changes in type I procollagen, thus reflecting, stoichiometrically, variations in levels of collagen synthesis.

Chronically stressed neonatal dermal human fibroblast cells were oxidatively stressed with 1 µM PMA and cells harvested at the given time points to measure changes in Procollagen-1 expression (T0, T6, T24, T48 and T72). A significant inhibition of Procollagen-1 synthesis (particularly at T6; ∼50%) was observed when directly compared to the control, untreated cells - see figure 8.

This suppression of Procollagen-1 synthesis is likely reflect an inability to synthesise new dermal matrix fibres in the skin.

### Discussion

To determine the effects of chronic stress on skin, we have subjected dermal fibroblasts to glucocorticoid treatment followed by acute oxidative stress, and measured changes in MMP-1, one of the most prominent dermal proteases with respect to skin ageing, and MMP-9, a key gelatinase released in the skin following acute doses of UVR. We have also measured the levels of procollagen type I C-peptide, which is an excellent indicator of the amount of collagen I synthesised. Our results demonstrate, for the first time, a significant inhibition of matrix removal (MMP-1, MMP-9) and repair (Procollagen I) in the chronically stressed skin. Thus, both matrix degradation and matrix synthesis are suppressed.

Together with our previous observations describing a significant exacerbation of inflammation in a chronically stressed skin (see Example 1) these data indicate that chronic stress plays a significant role in contributing to connective tissue damage (by impaired removal of degenerated collagen fibres and abnormal elastosis).

In conclusion, the identification of these skin-related markers, with respect to matrix remodelling and chronic stress, provides us with an excellent *in vitro* model in which to determine new routes of intervention to reduce the effects of stress on skin condition. We have used this model to identify agents that suppress the deleterious effects of chronic glucocorticoid exposure on skin cells, as will now be described in Example 3.

### Example 3 - Identification of agents that suppress glucocorticoid-mediated effects on skin cells - inflammation

### Materials and Methods

The same methods were used as described in Example 1, except that various test agents, as shown in Table 1, were added to the cells during the pre-treatment period.

Table 1: Summary of selected agents tested in chronically stressed Human Umbilical Vein Endothelial cells.

**Table 1**

| **Agent:** | **Source:** |
|---|---|
| 22OH-cholesterol | Sigma H9384 |
| Alliin | Aldrich 74264 |
| Caffeic acid | Sigma C0625 |
| Ciglitazone | Calbiochem 230950 |
| Commipheric acid | C.Rawlins, Unilever Research |
| | Colworth (method shown below) |
| Curcumin | Sigma C7727 |
| Geldanamycin | Sigma G3381 |
| Ginsenoside Rb1 | Sigma G0777 |
| Ginsenoside Rc | Sigma G0902 |
| Kyolic garlic | Quest Vitamins Ltd, Birmingham, UK |
| Mevinolin | Sigma M2147 |
| Okadaic acid | Sigma 09381 |
| Pycnogenol | Nature's Aid, Preston, UK |
| Resveratrol | Sigma R5010 |
| Rosemary extract | A&E Connock, Hampshire, UK |
| Theanine | Sigma E4393 |
| WY14643 | Calbiochem 681725 |

A vehicle control and a dexamethasone control were included in each assay.

### Methodology for extraction of Commipheric acid

330 g of guggul lipid was used as the starting material and 128g of product (42.6% yield) was generated. This contained 28.2% commipheric acid, as determined by gas chromatography. 100g of this product was further processed by silica treatment, using hexane/ethyl acetate ratios for separation. Silica treatment was conducted on a 750g silica 60 column using 80/20 v/v hexane/ethyl acetate. Although the saponified product would not dissolve completely in this mixture it was still applied to the column. The column was blocked when further 80/20 solvent was added, however, this cleared when a 60/40 ratio solvent was passed through.

The Thin Layer Chromatography (TLC) plate of the 60/40 fraction indicated that there was a higher concentration of components between the commipheric acid spots and the solvent front (high eluting components) than was found in the standard, a previously generated commipheric acid-rich fraction known to be 70% pure. The 50/50 sample contained high levels of components nearer to the baseline (low eluting components) and was discarded. The overall yield from the first silica treatment was 34.7g (14.7%) in the 60/40 solvent and 3.2% in the 50/50 solvent.

The 60/40 extract from the first silica treatment was further purified by a second round of silica treatment, but this time using a 150g silica column. No solubility issues were encountered during this silica column treatment. The samples were evaporated and the fractions collected and analysed. The TLC plate showed that the 60/40 fraction contains more high eluting material than the 70% commipheric acid standard. This is because its removal requires the 80/20 solvent. It also shows that the 50/50 fraction and, more so, the column remnants contain lower eluting material. After the second silica treatment, 92% of the concentrate added to the column was collected, 23.5g or 10% overall from the 60/40 fraction, 8.3g or 3.4% from the 80/20 fraction. In addition, the 60/40 fraction was analysed and found to be 77.9% commipheric acid by GC, the 80/20 fraction 49% commipheric acid.

### Results

Endothelial cells that had been chronically stressed with a synthetic glucocorticoid (1 µM Dexamethasone) for five days, were oxidatively stressed with 1 µM PMA for 24 hours. During this period, ICAM-1 synthesis was assayed as a measure of inflammatory response. Potential actives (shown in Table 1) were added to the cells during the pre-treatment period.

Any agents which showed significant increases in LDH (i.e. were cytotoxic) were discarded from this study. No notable changes in cellular morphology or cell number were observed throughout these investigations.

The results are shown in Table 2. Those agents which suppressed ICAM-1 synthesis in chronically stressed endothelial cells are discussed in more detail below. Those agents which did not suppress ICAM-1 synthesis at the concentration shown, can not be discounted completely at this stage, as dose responses have not been performed. Mostly, maximum, upper concentrations were employed throughout this study (with no signs of toxicity).

Table 2:

**Table 2**

| **Agent:** | **Conc:** | Effective? | **Role?** |
|---|---|---|---|
| 22-OH-cholesterol | 1 µM | Yes | LXR activator |
| Alliin | 10 µM | No | Antioxidant |
| Caffeic acid | 150 µM | No | Antioxidant |
| Ciglitazone | 10 µM | Yes | PPAR gamma activator |
| Commipheric acid | 10 µg/ml | Yes | PPAR gamma activator |
| Curcumin | 1 µM | Yes | AP-1 inhibitor |
| Geldanamycin | 0.1 µM | No | modulate GC binding |
| Geldanamycin | 1 µM | No | modulate GC binding |
| Ginsenoside Rb1 | 1 µM | Yes | cortisol suppressor |
| Ginsenoside Rc | 1 µM | Yes | cortisol suppressor |
| Kyolic garlic | 10 µg/ml | No | reduce oxidative stress |
| Mevinolin | 1 µM | Yes | PXR activator |
| Okadaic acid | 1 nM | Yes | Phosphatase inhibitor |
| Pycnogenol | 10 µg/ml | No | NF-κB & ICAM inhibitor |
| Resveratrol | 10 µM | No | anti-inflammatory |
| Rosemary extract | 1% | No | Antioxidant |
| Theanine | 10 µM | No | free radical scavenger |
| WY14643 | 10 µM | No | anti-inflammatory |

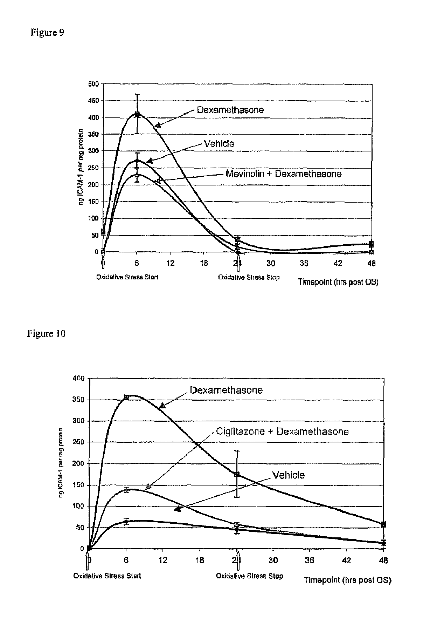

**Mevinolin**: when this agent was added routinely to the HUVECs in the presence of dexamethasone, a significant inhibition in ICAM-1 expression was observed. These suppressed levels of ICAM-1 are almost identical to control, untreated cells just below that induced by PMA in cells pre-treated with vehicle alone - see Figure 9.

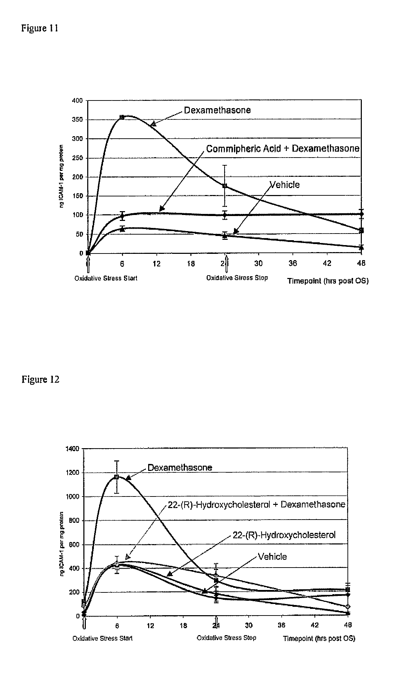

**Ciglitazone and Commipheric acid**, both PPAR gamma agonists, inhibited the increased synthesis of ICAM-1 induced by oxidative stress with PMA in endothelial cells chronically treated with dexamethasone. The level of ICAM-1 secreted by the GC-treated cells was almost brought back down to that induced by PMA in cells pre-treated with vehicle alone - see Figures 10 and 11.

**Ginseng, Rc and Rb1 fractions:** both fractions inhibited the increased synthesis of ICAM-1 induced by oxidative stress with PMA in endothelial cells chronically treated with dexamethasone. Ginsenoside Rc proved to be more effective than Ginseng Rb1 - data not shown.

**Curcumin:** this JNK/AP-1 inhibitor was shown to inhibit the increased synthesis of ICAM-1 induced by oxidative stress with PMA in endothelial cells chronically treated with dexamethasone. The levels of ICAM-1 secreted by the treated cells was almost brought back down to that induced by PMA in cells pre-treated with vehicle alone - data not shown.

**22-(R)-Hydroxycholesterol:** this oxysterol LXR ligand demonstrated a significant inhibition of increased synthesis of ICAM-1 induced by oxidative stress with PMA in endothelial cells chronically treated with dexamethasone. The levels of ICAM-1 secreted by the treated cells was almost brought back down to that induced by PMA in cells pre-treated with vehicle alone - see Figure 12.

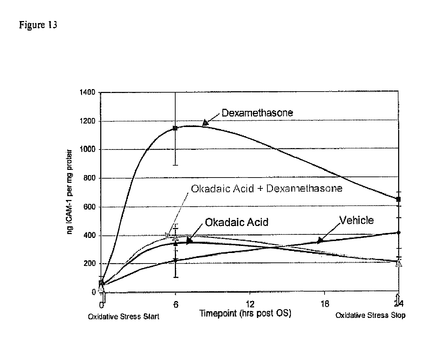

**Okadaic acid:** this polyether marine natural product is an effective serine and threonine phosphatase inhibitor. In this study, we have added okadaic acid in the presence of dexamethasone and observed a significant inhibition of the increased synthesis of ICAM-1 induced by oxidative stress with PMA in endothelial cells chronically treated with dexamethasone. The levels of ICAM-1 secreted by the treated cells was almost brought back down to that induced by PMA in cells pre-treated with vehicle alone - see Figure 13.

### Discussion

The most effective intervention agents in this study have included a selection of activators of a number of nuclear orphan receptors (PXR, LXR, PPAR-γ), with anti-inflammatory properties. In addition, nutritional compounds which actively suppress key transcription factors (like NF-κB and AP-1) proved to be good suppressors of ICAM-1 expression. One other final class of inhibitory agents employed in this *in vitro* screening model, was the polyether marine natural product, okadaic acid, which has been shown to inhibit serine and threonine-like phosphatases. Active inhibition through this mode of action may provide a new route of intervention to prevent the stress-induced accumulation of transcriptionally active glucocorticoids.

### Example 4 - Identification of agents that suppress alucocorticoid-mediated effects on skin cells - matrix remodelling

The same methods were used as described in Example 2, except that various test agents were added to the cells during the pre-treatment period.

### Methodology for extraction of Wolfberry

The dried, powdered plant material (∼10g) was placed into a thimble, 200 ml acetone was added to the reservoir and the apparatus heated. This solution was refluxed with boiling chips for 16 hours, then acetone evaporated under nitrogen. The final residue was stored at -20°C under nitrogen.

### Results

It was found that ciglitazone, activin (Powerherbs, Power Health Product Ltd., York, UK), shiitake extract (Bio-Support, Worcester, UK), wolfberry extract (K. Hunter, Unilever Research Colworth, UK - see above), boswellia extract (Alchem International Ltd., New Delhi, India), curcumin, commipheric acid, ginseng Rb1 fraction and ginseng Rc fraction all inhibited the effects of glucocorticoid on levels of MMP-1 and procollagen expression following PMA-induced acute stress.

The various features and embodiments of the present invention, referred to in individual sections above apply, as appropriate, to other sections, *mutatis mutandis.* Consequently features specified in one section may be combined with features specified in other sections, as appropriate.

All publications mentioned in the above specification are herein incorporated by reference. Various modifications and variations of the described methods and products of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are apparent to those skilled in the relevant fields are intended to be within the scope of the following claims.

## Claims

1. Use of a composition capable of inhibiting glucocorticoid-induced chronic stress in a dermal cell or a cell involved in skin inflammatory responses in the manufacture of a composition for use in reducing the effects of psychologically-mediated stress on the skin of a human or animal,
wherein the composition comprises a first substance which is capable of inhibiting glucocorticoid-induced chronic stress in a cell involved in skin inflammatory responses and a second substance which is capable of inhibiting glucocorticoid-induced chronic stress in a dermal cell,
wherein the composition comprises a first substance which is ginseng Rb1, and a second substance which is commipheric acid.

2. Use according to claim 1 wherein the composition is administered orally.

3. Use according to claim 1 wherein the composition is administered topically.

4. A method of reducing the effects of psychologically-mediated stress on the skin of a human or animal which method comprises administering to the individual a composition capable of inhibiting glucocorticoid-induced chronic stress in a dermal cell or a cell involved in skin inflammatory responses, wherein the composition comprises a first substance which is capable of inhibiting glucocorticoid-induced chronic stress in a cell involved in skin inflammatory responses and a second substance which is capable of inhibiting glucocorticoid-induced chronic stress in a dermal cell,
wherein the composition comprises a first substance which is ginseng Rb1, and a second substance which is commipheric acid.

5. A method according to claim 4 wherein the composition is administered orally.

6. A method according to claim 4 wherein the composition is administered topically.

7. A composition comprising a first substance which is ginseng Rb1 and a second substance which is commipheric acid.

8. A composition according to claim 7 which is a nutritional supplement.

9. A composition according to claim 7 which is a cosmetic composition.
